(19) Europäisches Patentamt
European Patent Office
Office européen des brevets

(11) Veröffentlichungsnummer: **0 126 234**
**A2**

# (12) EUROPÄISCHE PATENTANMELDUNG

(21) Anmeldenummer: **84102960.6**

(22) Anmeldetag: **17.03.84**

(51) Int. Cl.³: **C 07 D 285/06**, A 01 N 47/40

(30) Priorität: **20.05.83 DE 3319008**

(43) Veröffentlichungstag der Anmeldung: **28.11.84**
**Patentblatt 84/48**

(84) Benannte Vertragsstaaten: **BE CH DE FR GB IT LI LU NL SE**

(71) Anmelder: **SCHERING AKTIENGESELLSCHAFT Berlin und Bergkamen,**
**Müllerstrasse 170/178 Postfach 65 03 11,**
**D-1000 Berlin 65 (DE)**

(72) Erfinder: **Krüger, Hans-Rudolf, Dr., Kulmbacher Strasse 15, D-1000 Berlin-30 (DE)**
Erfinder: **Krähmer, Hansjörg, Dr., Fürstendamm 10A, D-1000 Berlin 28 (DE)**
Erfinder: **Rusch, Reinhardt, Dr., Wildkanzelweg 29, D-1000 Berlin 28 (DE)**
Erfinder: **Sjut, Volkert, Dr., Karmeliterweg 22, D-1000 Berlin 28 (DE)**

(54) **1,2,3-Thiadiazol-3-in-5-yliden-Harnstoffderivate, Verfahren zur Herstellung dieser Verbindungen sowie diese enthaltende Mittel mit wuchsregulatorischer und entblätternder Wirkung.**

(57) Die Erfindung betrifft neue 1,2,3-Thiadiazol-3-in-5-yliden-harnstoffderivate der allgemeinen Formel

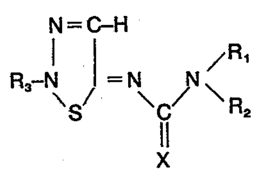

I

in der

$R_1$ Wasserstoff oder einen gegebenenfalls ein- oder mehrfach durch Sauerstoff- oder Schwefelatome unterbrochenen $C_1$–$C_4$-Alkylrest,

$R_2$ einen gegebenenfalls ein- oder mehrfach durch Sauerstoff- oder Schwefelatome unterbrochenen $C_1$–$C_4$-Alkylrest, einen gegebenenfalls ein- oder mehrfach durch Alkyl substituierten $C_3$–$C_8$-Cycloalkylrest, einen gegebenenfalls ein- oder mehrfach durch Alkyl und/oder Halogen und/oder Alkylthio und/oder Alkoxy und/oder Trifluormethyl und/oder die Nitrogruppe substituierten aromatischen Kohlenwasserstoffrest oder einen gegebenenfalls substituierten heterocyclischen, mindestens ein N-Atom enthaltenen, Kohlenwasserstoffrest oder $R_1$ und $R_2$ gemeinsam mit dem N-Atom die Morpholino-, Piperidino- oder Pyrrolidinogruppe,

$R_3$ einen gegebenenfalls substituierten $C_1$–$C_{10}$-Alkylrest, einen $C_2$–$C_6$-Alkenyl- oder $C_3$–$C_6$-Alkinylrest oder einen gegebenenfalls substituierten Aryl-$C_1$–$C_2$-alkylrest und

X ein Sauerstoff- oder ein Schwefelatom

bedeuten sowie deren Säureadditionssalze mit anorganischen und organischen Säuren, Verfahren zur Herstellung dieser Verbindungen sowie diese enthaltende Mittel mit wuchsregulatorischer und entblätternder Wirkung.



ACTORUM AG

Schering Aktiengesellschaft
Gewerblicher Rechtsschutz

SCHERING

Die Erfindung betrifft neue 1,2,3-Thiadiazol-3-in-5-yliden-harnstoffderivate, Verfahren zur Herstellung dieser Verbindungen sowie diese enthaltende Mittel mit wuchsregulatorischer und entblätternder Wirkung.

1,2,3-Thiadiazol-harnstoffderivate mit wuchsregulatorischer und entblätternder Wirkung sind bereits bekannt (DE-OS 2214632; DE-OS 2506690). Obwohl sich Produkte dieser Art in der Praxis bewährt haben, besteht weiterhin ein Bedürfnis nach Verbindungen mit noch größerer Wirkungsintensität und Wirkungsgeschwindigkeit.

Aufgabe der vorliegenden Erfindung ist die Schaffung neuer 1,2,3-Thiadiazol-harnstoff-derivate, welche strukturanaloge Verbindungen gleicher Wirkungsrichtung bezüglich ihrer Wirkungsintensität und Wirkungsgeschwindigkeit übertreffen.

Diese Aufgabe wird erfindungsgemäß gelöst durch 1,2,3-Thiadiazol-3-in-5-yliden-harnstoffderivate der allgemeinen Formel

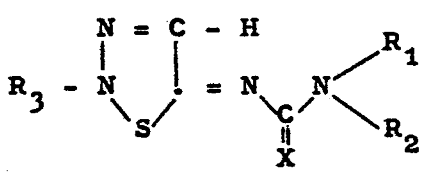

I

in der

$R_1$ Wasserstoff oder einen gegebenenfalls ein- oder mehrfach durch Sauerstoff- oder Schwefelatome unterbrochenen $C_1$-$C_4$-Alkylrest,

$R_2$ einen gegebenenfalls ein- oder mehrfach durch Sauerstoff- oder Schwefelatome unterbrochenen $C_1$-$C_4$-Alkylrest, einen gegebenenfalls ein- oder mehrfach durch Alkyl substituierten $C_3$-$C_8$-Cycloalkylrest, einen gegebenenfalls ein- oder mehrfach durch Alkyl und/oder Halogen und/oder Alkylthio und/oder Alkoxy und/oder Trifluormethyl und/oder die Nitrogruppe substituierten aromatischen Kohlenwasserstoffrest oder einen gegebenenfalls substituierten heterocyclischen, mindestens ein N-Atom enthaltenden, Kohlen-

-2-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101, Bankleitzahl 100 100 10

wasserstoffrest oder $R_1$ und $R_2$ gemeinsam mit dem N-Atom die Morpholino-, Piperidino- oder Pyrrolidinogruppe,

$R_3$ einen gegebenenfalls substituierten $C_1$-$C_{10}$-Alkylrest, einen $C_2$-$C_6$-Alkenyl- oder $C_3$-$C_6$-Alkinylrest oder einen gegebenenfalls substituierten Aryl-$C_1$-$C_2$-alkylrest und

X ein Sauerstoff- oder ein Schwefelatom

bedeuten sowie deren Säureadditionssalze mit anorganischen und organischen Säuren.

Die erfindungsgemäßen Verbindungen eignen sich hervorragend zur Entblätterung von Pflanzen, vorzugsweise von Baumwollpflanzen, und übertreffen hier in überraschender Weise bekannte Mittel analoger Konstitution.

Außer in Baumwollkulturen können die Verbindungen vorteilhafterweise auch in Baumschulen, Obstkulturen und Leguminosen angewandt werden. Die zu erntenden Pflanzen oder Pflanzenteile werden hierdurch in vorteilhafterweise Weise sowohl leichter zugänglich gemacht als auch in ihrer Reife erheblich beschleunigt. Bei entsprechenden Umweltbedingungen bilden so behandelte Pflanzen später wieder gesundes,normales Blattwerk aus.

Die erfindungsgemäßen Verbindungen entfalten außerdem eine charakteristische Cytokinin-Wirkung, worin sie bekannte Cytokinine überraschenderweise ebenfalls übertreffen.

Die erfindungsgemäßen Verbindungen eignen sich daher weiterhin hervorragend zur Regulierung des Pflanzenwachstums bei verschiedenen Kulturpflanzen.

Die erfindungsgemäßen Verbindungen vermögen das vegetative Wachstum von Kulturpflanzen zu fördern, in gewissen Konzentrationsbereichen aber auch zu hemmen. Darüberhinaus ist es möglich,

-3-

gewisse Mehrerträge durch die Beeinflussung der generativen Phase zu erreichen.

Unter bestimmten Bedingungen können sie sogar eine Anti-Streß-Wirkung entfalten.

Da die erfindungsgemäßen Verbindungen sowohl qualitative und quantitative Veränderungen von Pflanzen als auch Veränderungen im Metabolismus in den Pflanzen verursachen, sind sie in die Klasse der Pflanzenwachstumsregulatoren einzustufen, die sich durch folgende Anwendungsmöglichkeiten auszeichnen:

Hemmung des vegetativen Wachstums bei holzigen und krautigen Pflanzen zum Beispiel an Straßenrändern, Gleisanlagen und anderen, um ein zu üppiges Wachstum zu unterbinden. Wuchshemmung beim Getreide, um das Lagern oder Umknicken zu unterbinden, bei Baumwolle zur Ertragserhöhung.

Beeinflussung der Verzweigung von vegetativen und generativen Organen bei Zier- und Kulturpflanzen zur Vermehrung des Blütenansatzes oder bei Tabak und Tomate zur Hemmung von Seitentrieben.

Verbesserung der Fruchtqualität, zum Beispiel eine Zuckergehaltssteigerung beim Zuckerrohr, bei Zuckerrüben oder bei Obst und eine gleichmäßigere Reife des Erntegutes, die zu höheren Erträgen führt.

Erhöhung der Widerstandskraft gegen Streß, so zum Beispiel gegen klimatische Einflüsse, wie Kälte und Trockenheit, aber auch gegen phytotoxische Einflüsse von Chemikalien.

Beeinflussung des Latexflusses bei Gummipflanzen.

Ausbildung parthenokarper Früchte, Pollensterilität und Geschlechtsbeeinflussung sind ebenfalls Anwendungsmöglichkeiten.

-4-

. Kontrolle der Keimung von Samen oder des Austriebes von Knospen.

Entlaubung oder Beeinflussung des Fruchtfalles zur Ernteerleichterung.

Die erfindungsgemäßen Verbindungen eignen sich insbesondere zur Beeinflussung des vegetativen und generativen Wachstums bei einigen Leguminosen, wie zum Beispiel Soja, und bei Beta-Rüben.

Die Aufwandmengen betragen je nach Anwendungsziel im allgemeinen von 0,001 bis 1 kg Wirkstoff/ha, gegebenenfalls können auch höhere Aufwandmengen eingesetzt werden.

Die Anwendungszeit richtet sich nach dem Anwendungsziel und den klimatischen Bedingungen.

In den durch die allgemeine Formel I gekennzeichneten Verbindungen können zum Beispiel bedeuten:

$R_1$ Wasserstoff, $C_1$-$C_4$-Alkyl, zum Beispiel Methyl, Äthyl, Propyl, Isopropyl oder Butyl;

$R_2$ $C_1$-$C_4$-Alkyl, zum Beispiel Methyl, Äthyl oder Propyl, Cycloalkyl mit 5 bis 8 Kohlenstoffatomen, zum Beispiel Cyclopentyl, Cyclohexyl, Methylcyclohexyl; Aryl, wie zum Beispiel Phenyl, Halophenyl, $C_1$-$C_4$-Alkylphenyl, $C_1$-$C_4$-Alkoxyphenyl, wie zum Beispiel Methoxyphenyl, Nitrophenyl, Trifluormethylphenyl oder 2-Pyridyl.

Unter den in der allgemeinen Formel I mit $R_3$ bezeichneten Resten sind weiterhin beispielsweise zu verstehen als gegebenenfalls substituierte $C_1$-$C_{10}$-Alkylreste, Methyl, Äthyl, Propyl, Isopropyl, n-Butyl, Isobutyl, sec-Butyl, tert.-Butyl, Pentyl, Hexyl, Heptyl, Octyl, Nonyl, Decyl, 3,3-Dimethylpropyl, Chlormethyl, Fluormethyl, 2-Chloräthyl, 2-Hydroxyäthyl, 2-Methylsulfonyloxyäthyl, 2-Acetoxyäthyl,



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Methoxymethyl, 2-Methoxyäthyl, 2-Äthoxyäthyl, 2-Phenoxyäthyl, 2-(2,4-Dichlorphenoxy)-äthyl, 2-(4-Chlorphenoxy)-äthyl, 2-Dimethylamino-äthyl, 3-Chlorpropyl, 3-Methoxypropyl, (2-Methyl-1,3-dioxolan-2-yl)-methyl, 3-Dimethylaminopropyl, 3-Phenoxypropyl, 2,2-Dichlorcyclopropylmethyl; als $C_2$-$C_6$-Alkenylreste, Äthenyl, 2-Propenyl, 3-Methyl-2-buten-1-yl, 2-Methyl-1-propen-3-yl, Hexenyl, Heptenyl, Octenyl;
als $C_3$-$C_6$-Alkinylreste, 2-Propinyl, Butinyl, Pentinyl, Hexinyl;
als aliphatisch-aromatische Kohlenwasserstoffreste Benzyl, 2-Fluorbenzyl, 3-Fluorbenzyl, 4-Fluorbenzyl, 2-Chlorbenzyl, 4-Chlorbenzyl, 2-Brombenzyl, 3-Brombenzyl, 4-Brombenzyl, 2,4-Dichlorbenzyl, 2,6-Dichlorbenzyl, 3,4-Dichlorbenzyl, 2-Methylbenzyl, 3-Methylbenzyl, 4-Methylbenzyl, 2-Nitrobenzyl, 3-Nitrobenzyl, 4-Nitrobenzyl, 2-Trifluormethylbenzyl, 3-Trifluormethylbenzyl, 4-Trifluormethylbenzyl, 2-Methoxybenzyl, 3-Methoxybenzyl, 4-Methoxybenzyl, 2-Äthoxybenzyl, 3-Äthoxybenzyl, 4-Äthoxybenzyl, 2-Propoxybenzyl, 3-Propoxybenzyl, 4-Propoxybenzyl, 2-Butoxybenzyl, 3-Butoxybenzyl, 4-Butoxybenzyl, 2-Methylthiobenzyl, 3-Methylthiobenzyl, 4-Methylthiobenzyl, 2-Äthylthiobenzyl, 3-Äthylthiobenzyl, 4-Äthylthiobenzyl, 2-Butylthiobenzyl, 3-Butylthiobenzyl, 4-Butylthiobenzyl.

X stellt ein Sauerstoff- oder ein Schwefelatom dar.

Als anorganische und organische Säure zur Bildung der Säureadditionssalze sind beispielsweise zu nennen, die Halogenwasserstoffsäuren, wie zum Beispiel die Chlorwasserstoffsäure und die Bromwasserstoffsäure, ferner Phosphorsäure, Schwefelsäure und Salpetersäure, mono- und bifunktionelle Carbonsäuren und Hydroxycarbonsäuren, wie zum Beispiel Essigsäure, Maleinsäure, Bernsteinsäure, Fumarsäure, Weinsäure, Zitronensäure, Salicylsäure, Sorbinsäure, Milchsäure sowie Sulfonsäuren, wie zum Beispiel p-Toluolsulfonsäure und 1,5-Naphthalindisulfonsäure.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Diese Säureadditionssalze können nach den üblichen Salzbildungsverfahren, zum Beispiel durch Lösen einer Verbindung der Formel I in einem geeigneten Lösungsmittel und Hinzufügen der Säure erhalten werden.

Von den erfindungsgemäßen Verbindungen zeichnen sich durch eine wuchsregulatorische und entblätternde Wirkung insbesondere diejenigen aus, bei denen in der allgemeinen Formel I der Rest

$R_1$ Wasserstoff oder Methyl,
$R_2$ Phenyl, 2-Pyridyl und
$R_3$ Methyl, Äthyl, Propyl, Isopropyl, Butyl, Pentyl, und Hexyl darstellen.

Von herausragender Wirkung sind die folgenden erfindungsgemäßen Verbindungen:

3-(2-Methyl-1,2,3-thiadiazol-3-in-5-yliden)-1-phenylharnstoff
3-(2-Äthyl-1,2,3-thiadiazol-3-in-5-yliden)-1-phenylharnstoff, Hydrochlorid
3-(2-Äthyl-1,2,3-thiadiazol-3-in-5-yliden)-1-phenylharnstoff
3-(2-Propyl-1,2,3-thiadiazol-3-in-5-yliden)-1-phenylharnstoff
3-(2-Butyl-1,2,3-thiadiazol-3-in-5-yliden)-1-phenylharnstoff
3-(2-Methyl-1,2,3-thiadiazol-3-in-5-yliden)-1-phenylharnstoff, Hydrochlorid
3-(2-Propyl-1,2,3-thiadiazol-3-in-5-yliden)-1-phenylharnstoff, Hydrochlorid
3-(2-Butyl-1,2,3-thiadiazol-3-in-5-yliden)-1-phenylharnstoff, Hydrochlorid
3-(2-Isopropyl-1,2,3-thiadiazol-3-in-5-yliden)-1-phenylharnstoff
3-(2-Äthenyl-1,2,3-thiadiazol-3-in-5-yliden)-1-phenylharnstoff

-7-

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden.

Sofern eine Verbreiterung des Wirkungsspektrums beabsichtigt ist, können auch andere Biozide zugesetzt werden. Beispielsweise eignen sich als herbizid wirksame Mischungspartner diejenigen Wirkstoffe, die in Weed Abstracts, Vol. 31, No. 7, 1982, unter dem Titel "Lists of common names and abbreviations employed for currently used herbicides and plant growth regulators in weed abstracts" aufgeführt sind. Außerdem können auch nicht phytotoxische Mittel angewendet werden, die mit Herbiziden und/oder Wuchsregulatoren eine synergistische Wirkungssteigerung ergeben können, wie unter anderem Netzmittel, Emulgatoren, Lösungsmittel und ölige Zusätze.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Netz-, Haft-, Emulgier- und/oder Dispergierhilfsmitteln angewandt.

Geeignete flüssige Trägerstoffe sind zum Beispiel Wasser, aliphatische und aromatische Kohlenwasserstoffe, wie Benzol, Toluol, Xylol, Cyclohexanon, Isophoron, Dimethylsulfoxyd, Dimethylformamid, weiterhin Mineralölfraktionen und Pflanzenöle.

-8-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101, Bankleitzahl 100 100 10

Als feste Trägerstoffe eignen sich Mineralerden, zum Beispiel Tonsil, Silicagel, Talkum,Kaolin, Attapulgit, Kalkstein, Kieselsäure und pflanzliche Produkte, zum Beispiel Mehle.

An oberflächenaktiven Stoffen sind zu nennen: zum Beispiel Calciumligninsulfonat, Polyoxyäthylenalkylphenyl-äther, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Sofern die Wirkstoffe zur Saatgutbeize Verwendung finden sollen, können auch Farbstoffe zugemischt werden, um dem gebeizten Saatgut eine deutlich sichtbare Färbung zu geben.

Der Anteil des bzw. der Wirkstoffe(s) in den verschiedenen Zubereitungen kann in weiten Grenzen variieren. Beispielsweise enthalten die Mittel etwa 10 bis 90 Gewichtsprozent Wirkstoffe, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen von etwa 100 bis 1000 Liter/ha. Eine Anwendung der Mittel im sogenannten "Low-Volume" oder "Ultra-Low-Volume-Verfahren" ist ebenso möglich wie ihre Applikation in Form von sogenannten Mikrogranulaten.

Zur Herstellung der Zubereitungen werden zum Beispiel die folgenden Bestandteile eingesetzt:



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

A. SPRITZPULVER

a) 80 Gewichtsprozent Wirkstoff
15 Gewichtsprozent Kaolin
5 Gewichtsprozent oberflächenaktive Stoffe auf Basis des Natriumsalzes des N-Methyl-N-oleyl-taurins und des Calciumsalzes der Ligninsulfonsäure

b) 50 Gewichtsprozent Wirkstoff
40 Gewichtsprozent Tonmineralien
5 Gewichtsprozent Zellpech
5 Gewichtsprozent oberflächenaktive Stoffe auf der Basis einer Mischung des Calciumsalzes der Ligninsulfonsäure mit Alkylphenolpolyglykoläther

c) 20 Gewichtsprozent Wirkstoff
70 Gewichtsprozent Tonmineralien
5 Gewichtsprozent Zellpech
5 Gewichtsprozent oberflächenaktive Stoffe auf der Basis einer Mischung des Calciumsalzes der Ligninsulfonsäure mit Alkylphenolpolyglykoläthern

d) 5 Gewichtsprozent Wirkstoff
80 Gewichtsprozent Tonsil
10 Gewichtsprozent Zellpech
5 Gewichtsprozent oberflächenaktive Stoffe auf der Basis eines Fettsäurekondensationsproduktes

B. EMULSIONSKONZENTRAT

20 Gewichtsprozent Wirkstoff
40 Gewichtsprozent Xylol
35 Gewichtsprozent Cyclohexanon
5 Gewichtsprozent Mischung von Nonylphenylpolyoxyäthylen oder Calciumdodecylbenzolsulfonat



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

C. PASTE

45 Gewichtsprozent Wirkstoff
5 Gewichtsprozent Natriumaluminiumsilikat
15 Gewichtsprozent Cetylpolyglykoläther mit 8 Mol Äthylenoxid
2 Gewichtsprozent Spindelöl
10 Gewichtsprozent Polyäthylenglykol
23 Teile Wasser

Die neuen, bisher nicht in der Literatur beschriebenen, erfindungsgemäßen Verbindungen können zum Beispiel hergestellt werden, indem man

A) Metallverbindungen der allgemeinen Formel

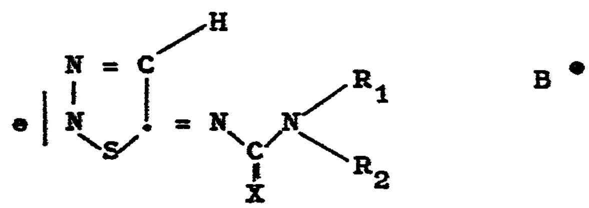

II

mit Verbindungen der allgemeinen Formel

$R_3 - Z$ III,

gegebenenfalls in Gegenwart eines Lösungsmittels und gegebenenfalls in Gegenwart eines Katalysators, reagieren läßt oder

B) (1,2,3-Thiadiazol-5-yl)-harnstoffe der allgemeinen Formel

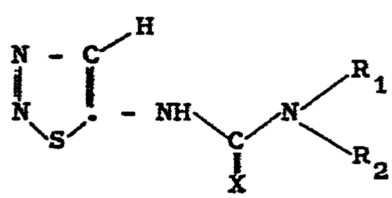

IV

mit Verbindungen der allgemeinen Formel

$R_3 - Z$ III



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

gegebenenfalls in Gegenwart von säurebindenden Mitteln, sowie gegebenenfalls in Gegenwart eines geeigneten Katalysators umsetzt, worin $R_1$, $R_2$, $R_3$ und X die oben genannte Bedeutung haben, Z ein Halogenatom oder den Rest $R_3OSO_2$-O und B ein einwertiges Metalläquivalent bedeuten.

Als Halogen ist beispielsweise zu nennen, Chlor, Brom oder Jod; als Alkyloxysulfonyloxy-Reste eignen sich die Methyl, Äthyl und die Propyloxysulfonyloxy-Gruppe. Geeignete einwertige Metalläquivalente sind vorzugsweise ein Natrium- oder Lithiumatom.

Die Reaktion B kann sowohl gegebenenfalls in Gegenwart eines Lösungsmittels sowohl mit einem Überschuß an Alkylierungsagenz $R_3$-Z ohne säurebindende Mittel, als auch unter Zusatz einer starken Base durchgeführt werden.

Wird auf den Zusatz von Base verzichtet, so erhält man die erfindungsgemäßen Verbindungen direkt in Form ihrer Additionssalze, aus denen wiederum durch Verwendung starker Basen die erfindungsgemäßen Verbindungen der Formel I freigesetzt werden können.

Die Umsetzung der Reaktionspartner erfolgt zwischen -10 und 150°C, im allgemeinen jedoch zwischen Raumtemperatur und Rückflußtemperatur des entsprechenden Reaktionsgemisches.

Die Reaktionsdauer beträgt 1 bis 72 Stunden. In der Regel erfolgen die Reaktionen bei Normaldruck oder leichtem Überdruck. Zur Synthese der erfindungsgemäßen Verbindungen werden die Reaktanden in etwa äquimolaren Mengen eingesetzt. Geeignete Reaktionsmedien sind gegenüber den Reaktanden inerte Lösungsmittel. Die Wahl der Lösungs- bzw. Suspensionsmittel richtet sich nach dem Einsatz der entsprechenden Alkylhalogenide sowie Dialkylsulfate und der angewandten Säureakzeptoren. Als Lösungs- bzw. Suspensionsmittel seien beispielsweise genannt:



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101, Bankleitzahl 100 100 10

aliphatische und aromatische Kohlenwasserstoffe, wie Petroläther, Cyclohexan, Hexan, Heptan, Benzol, Toluol, Xylole; halogenierte Kohlenwasserstoffe wie Methylenchlorid, Äthylenchlorid, Chlorbenzol, Chloroform, Tetrachlorkohlenstoff, Tetrachloräthylen; Äther wie Diäthyläther, Diisopropyläther, Anisol, Dioxan, Tetrahydrofuran, Äthylenglykoldiäthyläther, Diäthylenglykoldiäthyläther; Carbonsäurenitrile wie Aceto - nitril, Propionitril; Carbonsäureamide wie Dimethylformamid, Dimethylacetamid, N-Methylpyrrolidon, Tetramethylharnstoff; Dimethylsulfoxid; Ketone wie Aceton, Diäthylketon, Methyläthylketon; Alkohole wie Methanol, Äthanol, Propanol, Butanol und Gemische solcher Lösungsmittel untereinander.

In einigen Fällen kann auch der Reaktionspartner selbst als Lösungsmittel dienen.

Als Säureakzeptoren eignen sich organische Basen, wie zum Beispiel Triäthylamin, Trimethylamin, N,N-Dimethylanilin, Pyridin und Pyridinbasen (4-Dimethylaminopyridin) oder anorganische Basen wie Oxide, Hydroxide, Hydride, Carbonate, Hydrogencarbonate und Alkoholate von Alkali- und Erdalaklimetall sowie Amide und Alkalisalze von Carbonsäuren (KOH, NaOH, $Na_2CO_3$, $CH_3COONa$, NaH, KH, LiH, $CaH_2$).
Flüssige Basen wie Pyridin können gleichzeitig als Lösungsmittel eingesetzt werden. Entstehender Halogenwasserstoff kann in manchen Fällen auch mittels Durchleiten von Inertgas, zum Peispiel Stickstoff, aus dem Reaktionsgemisch entfernt werden oder an Molekularsieb adsorbiert werden.
Die Gegenwart eines Reaktionskatalysators kann von Vorteil sein. Als Katalysatoren sind Kaliumjodid und Oniumverbindungen geeignet, wie quaternäre Ammonium-, Phosphonium-und Arsoniumverbindungen sowie Sulfoniumverbindungen. Ebenfalls geeignet sind Polyglykoläther, insbesondere cyclische, wie zum Beispiel 18-Krone-6 und tertiäre Amine, wie zum Beispiel Tributylamin. Bevorzugte Verbindungen sind quaternäre Ammoniumverbindungen, wie zum Beispiel Benzyltriäthylammoniumchlorid und Tetrabutylammoniumbromid.

-13-

Die nach oben genannten Verfahren hergestellten erfindungsgemäßen Verbindungen können nach den üblichen Verfahren aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion.
Ein erhöhter Reinheitsgrad kann in der Regel durch säulenchromatographische Aufreinigung sowie durch fraktionierte Kristallisation erhalten werden.

Bei Verwendung gängiger Alkylierungsverfahren erhält man in der Regel neben den erfindungsgemäßen Verbindungen in unterschiedlichem Umfang Nebenprodukte.
Durch geeignete Reaktionsbedingungen, gegebenenfalls mittels geeigneter Katalysatoren ist eine regioselektive Alkylierung im Sinne der gewünschten erfindungsgemäßen Verbindungen möglich.

Die erfindungsgemäßen Verbindungen stellen in der Regel fast farblose, geruchlose, kristalline Körper dar, die schwerlöslich in Wasser und aliphatischen Kohlenwasserstoffen sind, mäßig bis gut löslich in halogenierten Kohlenwasserstoffen wie Chloroform und Tetrachlorkohlenstoff; Ketonen wie Aceton, Carbonsäureamiden wie Dimethylformamid, Sulfoxiden wie Dimethylsulfoxid, Carbonsäurenitrilen wie Acetonitril und niederen Alkoholen wie Methanol und Äthanol.

Als Lösungsmittel zur Umkristallisation bieten sich insbesondere Tetrachlorkohlenstoff, Chloroform, Acetonitril und Toluol an.

Die Ausgangsprodukte zur Herstellung der erfindungsgemäßen Verbindungen sind an sich bekannt oder können nach an sich bekannten Verfahren hergestellt werden.

Die folgenden Beispiele erläutern die Herstellung der erfindungsgemäßen Verbindungen.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101, Bankleitzahl 100 100 10

BEISPIEL 1

3-(2-Propyl-1,2,3-thiadiazol-3-in-5-yliden)-1-phenylharnstoff

44,0 g (0,2 Mol) 1-Phenyl-3-(1,2,3-thiadiazol-5-yl)-harnstoff werden in 200 ml wasserfreiem Dimethylformamid gelöst und vorsichtig mit 8,7 g (0,2 Mol) einer 55 %igen Dispersion von Natriumhydrid in Öl versetzt. Während der Zugabe wird das Gemisch durch Kühlung auf 30°C gehalten, anschließend wird 45 Minuten bei Raumtemperatur nachgerührt, bis sich kein Wasserstoff mehr entwickelt. Dann wird innerhalb von 15 Minuten eine Lösung von 22,47 ml (0,23 Mol) n-Propyljodid in 400 ml Dimethylformamid zugetropft, wobei die Reaktionstemperatur 20°C nicht übersteigen soll. Nach Ende der Zugabe wird das Gemisch noch 6 Stunden bei Raumtemperatur nachgerührt. Darauf wird das Reaktionsgemisch vorsichtig auf 1000 ml Eiswasser gegeben; anschließend wird dreimal mit je 500 ml Methylenchlorid extrahiert. Die Methylenchloridextrakte werden mit einer Lösung aus 8,0 g Natriumhydroxid in 80 ml Wasser gewaschen, dann über Magnesiumsulfat getrocknet, filtriert und im Vakuum eingeengt.

Man erhält so 55 g schwach gelb gefärbte Kristalle.
Die weitere Reinigung erfolgt säulenchromatographisch (Mitteldruck) an Kieselgel (Eluens: Diisopropyläther/Essigester 60:40).
Man erhält fast farblose Kristalle, die aus Diisopropyläther umkristallisiert werden können.

| | | | | |
|---|---|---|---|---|
| Ausbeute: | 20,0 g = 38,2 % der Theorie<br>3-(2-Propyl-1,2,3-thiadiazol-3-in-5-yliden)-1-phenylharnstoff | | | |
| Fp.: | 95°C | | | |
| DC: | Laufmittel = Essigester $R_f$-Wert: 0,670 | | | |
| Analyse: | Berechnet | C 54,94% | H 5,36% | N 21,36% |
| | Gefunden | C 55,14% | H 5,49% | N 21,41% |



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 2

3-(2-Propyl-1,2,3-thiadiazol-3-in-5-yliden)-1-phenylharnstoff Hydrochlorid

6,76 g (0,026 Mol) 3-(2-Propyl-1,2,3-thiadiazol-3-in-5-yliden)-1-phenylharnstoff werden in 180 ml Aceton gelöst. Hierzu werden bei 0°C 8,6 ml (0,052 Mol) einer gesättigten ätherischen Chlorwasserstofflösung zugetropft.

Es wird noch 15 Minuten bei Raumtemperatur nachgerührt. Zur Vervollständigung der Fällung wird noch mit 30 ml Diisopropyläther versetzt. Die schwach gelb gefärbten Kristalle werden abgesaugt, mit 50 ml Diisopropyläther nachgewaschen und getrocknet.

Ausbeute: 6,4 g = 87,5 % der Theorie
3-(2-Propyl-1,2,3-thiadiazol-3-in-5-yliden)-1-phenylharnstoff, Hydrochlorid

Fp.: 188-190°C Zersetzung

DC: Laufmittel = Essigester $R_f$-Wert: 0,615

| | | | | |
|---|---|---|---|---|
| Analyse: | Berechnet | C 48,23% | H 5,06% | N 18,75% |
| | Gefunden | C 47,95% | H 5,12% | N 18,54% |

-16-

**BEISPIEL 3**

**3-(2-Äthyl-1,2,3-thiadiazol-3-in-5-yliden)-1-phenylharnstoff**

a) 11,0 g (0,05 Mol) 1-Phenyl-3-(1,2,3-thiadiazol-5-yl)-harnstoff werden in 200 ml trockenem Äthylenglykoldiäthyläther suspendiert. Zuerst werden 2,6 g (0,01 Mol) 18-Krone-6 hinzugegeben, dann versetzt man vorsichtig mit 2,4 g (0,05 Mol) einer 50 %igen Dispersion von Natriumhydrid in Öl, wobei die Reaktionstemperatur 30°C nicht übersteigen soll.

Anschließend wird noch zwei Stunden bei Raumtemperatur nachgerührt. Zu der inzwischen klaren Lösung wird innerhalb von 15 Minuten eine Lösung von 4,9 ml (0,06 Mol) Äthyljodid in 20 ml Äthylenglykoldiäthyläther bei 25°C getropft. Nach Ende der Zugabe wird noch 8 Stunden bei Raumtemperatur nachgerührt. Die fast klare Lösung wird im Vakuum bei 40°C eingedampft. Der Rückstand wird in 200 ml Methylenchlorid aufgenommen; die organische Phase dann mit einer Lösung aus 2 g Natriumhydroxid in 20 ml Wasser gewaschen. Der mit 100 ml Wasser gewaschene Methylenchloridextrakt wird über Magnesiumsulfat getrocknet, filtriert und im Vakuum eingeengt. Man erhält 13,0 g schwachgelbe Kristalle, die aus Diisopropyläther umkristallisiert werden.

| | | | | |
|---|---|---|---|---|
| Ausbeute: | 6,38 g = 51,5 % der Theorie 3-(2-Äthyl-1,2,3-thiadiazol-3-in-5-yliden)-1-phenylharnstoff | | | |
| Fp.: | 123-124°C | | | |
| ~~DC:~~ | ~~Laufmittel = Essigester~~ | ~~$R_f$-Wert: 0,610~~ | | |
| Analyse: | berechnet | C 53,21% | H 4,87% | N 22,56% |
| | gefunden | C 53,46% | H 5,11% | N 22,58% |



Postanschrift: Schering Aktiengesellschaft, Postfach 65 03 11, D-1000 Berlin 65 · Für Besucher: Berlin-Wedding, Müllerstraße 170-173 · Telegramme: Scheringchemie Berlin

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

b) 11,0 g (0,05 Mol) 1-Phenyl-3-(1,2,3-thiadiazol-5-yl)-harnstoff werden in einer Lösung aus 5,92 g (0,09 Mol) Kaliumhydroxid in 30 ml Wasser gelöst, Hierzu werden innerhalb von 30 Minuten bei ca. 30°C 9,82 ml Diäthylsulfat getropft. Das Reaktionsgemisch wird zuerst 30 Minuten bei Raumtemperatur nachgerührt. Dann noch 30 Minuten auf 60°C erhitzt. Anschließend wird dreimal mit je 200 ml Methylenchlorid extrahiert. Die über Magnesiumsulfat getrockneten, filtrierten Methylenchloridextrakte werden im Vakuum eingeengt. Man erhält 11,4 g gelbliche Kristalle, die aus Diisopropyläther umkristallisiert werden.

Ausbeute: 4,96 g = 40,0 % der Theorie
3-(2-Äthyl-1,2,3-thiadiazol-3-in-5-yliden)-1-phenylharnstoff

Fp.: 123-124°C

DC: Laufmittel = Essigester $R_f$-Wert: 0,610

In analoger Weise lassen sich die weiteren erfindungsgemäßen Verbindungen herstellen.

-18-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Beispiel Nr. | Name der Verbindungen | Physikalische Konstante |
|---|---|---|
| 4 | 3-(2-Methyl-1,2,3-thiadiazol-3-in-5-yliden)-1-phenylharnstoff | Fp.: 175-178 °C |
| 5 | 3-(2-(2,6-Dichlorbenzyl)-1,2,3-thiadiazol-3-in-5-yliden)-1-phenylharnstoff | Fp.: 177 °C |
| 6 | 3-[2-(4-Chlorbenzyl)-1,2,3-thiadiazol-3-in-5-yliden]-1-phenylharnstoff | Fp.: 154 °C (Zersetzung) |
| 7 | 3-[2-(2-Chlorbenzyl)-1,2,3-thiadiazol-3-in-5-yliden]-1-phenylharnstoff | Fp.: 146 °C |
| 8 | 3-[2-(2,4-Dichlorbenzyl)-1,2,3-thiadiazol-3-in-5-yliden]-1-phenylharnstoff | Fp.: 140-142 °C |
| 9 | 3-(2-Äthyl-1,2,3-thiadiazol-3-in-5-yliden)-1-phenylharnstoff Hydrochlorid | Fp.: 197 °C (Zersetzung) |
| 10 | 3-(2-Butyl-1,2,3-thiadiazol-3-in-5-yliden)-1-phenylharnstoff | Fp.: 91- 92 °C |
| 11 | 3-(2-Methyl-1,2,3-thiadiazol-3-in-5-yliden)-1-phenylharnstoff, Hydrochlorid | Fp.: 211 °C (Zersetzung) |
| 12 | 3-(2-Butyl-1,2,3-thiadiazol-3-in-5-yliden)-1-phenylharnstoff, Hydrochlorid | Fp.: 188-189 °C (Zersetzung) |
| 13 | 3-(2-Isopropyl-1,2,3-thiadiazol-3-in-5-yliden)-1-phenylharnstoff | Fp.: 126-127 °C |
| 14 | 3-[2-(2-Propenyl)-1,2,3-thiadiazol-3-in-5-yliden]-1-phenylharnstoff | Fp.: 131 °C |
| 15 | 1-Phenyl-3-[2-(2-propinyl)-1,2,3-thiadiazol-3-in-5-yliden]-harnstoff | Fp.: 157 °C |
| 16 | 3-(2-Benzyl-1,2,3-thiadiazol-3-in-5-yliden)-1-phenylharnstoff | Fp.: 141 °C |



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Beispiel Nr. | Name der Verbindungen | Physikalische Konstante |
|---|---|---|
| 17 | 3-(2-(2-Chloräthyl)-1,2,3-thiadiazol-3-in-5-yliden)-1-phenylharnstoff | Fp.:135°C |
| 18 | 3-(2-(2-Acetoxyäthyl)-1,2,3-thiadiazol-3-in-5-yliden)-1-phenylharnstoff | Fp.:93,5-94°C |
| 19 | 3-(2-Decyl-1,2,3-thiadiazol-3-in-5-yliden)-1-phenylharnstoff | Fp.: 78- 80°C |
| 20 | 3-(2-Äthenyl-1,2,3-thiadiazol-3-in-5-yliden)-1-phenylharnstoff | Fp.:166-167°C |
| 21 | 3-(2-Methoxymethyl-1,2,3-thiadiazol-3-in-5-yliden)-1-phenylharnstoff | Fp.: 88- 89°C |
| 22 | 3-(2-Äthyl-1,2,3-thiadiazol-3-in-5-yliden)-1-methyl-1-phenylharnstoff | $n_D^{20}$: 1,6602 |
| 23 | 1-Äthyl-3-(2-äthyl-1,23-thiadiazol-3-in-5-yliden)-1-phenylharnstoff | Fp.: 70-73°C |

Die folgenden Ausführungsbeispiele dienen zur Erläuterung der Anwendungsmöglichkeiten der erfindungsgemäßen Verbindungen, die in Form der oben angeführten Zubereitungen erfolgte.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101, Bankleitzahl 100 100 10

BEISPIEL 21

Junge Baumwollpflanzen im Stadium von 4 bis 6 entwickelten Laubblättern wurden mit nachstehend angegebenen Wirkstoffen behandelt (Wiederholung 4-fach). Die ausgebrachte Spritzbrühmenge entsprach 500 Liter/ha. Die Pflanzen wurden im Gewächshaus in der Regel bei 19 bis 22°C gehalten. Drei Wochen nach der Applikation wurde der Prozentsatz abgeworfener Blätter festgestellt. Die Ergebnisse sind der folgenden Tabelle zu entnehmen.

| Erfindungsgemäße Verbindungen | Dosis in g Wirkstoff/ha | Entblätterung in % |
|---|---|---|
| 3-(2-Methyl-1,2,3-thiadiazol-3-in-5-yliden)-1-phenylharnstoff | 500 | 90 |
| 3-(2-Äthyl-1,2,3-thiadiazol-3-in-5-yliden)-1-phenylharnstoff Hydrochlorid | 500 | 100 |
| 3-(2-Äthyl-1,2,3-thiadiazol-3-in-5-yliden)-1-phenylharnstoff | 500 | 100 |
| 3-(2-Propyl-1,2,3-thiadiazol-3-in-5-yliden)-1-phenylharnstoff | 500 | 100 |
| 3-(2-Butyl-1,2,3-thiadiazol-3-in-5-yliden)-1-phenylharnstoff | 500 | 100 |
| 3-(2-Methyl-1,2,3-thiadiazol-3-in-5-yliden)-1-phenylharnstoff Hydrochlorid | 500 | 95 |
| 3-(2-Propyl-1,2,3-thiadiazol-3-in-5-yliden)-1-phenylharnstoff Hydrochlorid | 500 | 95 |
| 3-(2-Butyl-1,2,3-thiadiazol-3-in-5-yliden)-1-phenylharnstoff Hydrochlorid | 500 | 90 |
| 3-(2-Isopropyl-1,2,3-thiadiazol-3-in-5-yliden)-1-phenylharnstoff | 500 | 100 |
| 3-(2-(2-Propenyl)-1,2,3-thiadiazol-3-in-5-yliden)-1-phenylharnstoff | 500 | 80 |
| 1-Phenyl-3-(2-(2-propinyl)-1,2,3-thiadiazol-3-in-5-yliden)-1-phenylharnstoff | 500 | 75 |

-21-

| Erfindungsgemäße Verbindungen | Dosis in g Wirkstoff/ha | Entblätterung in % |
|---|---|---|
| 3-(2-(2-Chloräthyl)-1,2,3-thiadiazol-3-in-5-yliden)-1-phenylharnstoff | 500 | 75 |
| 3-(2-Äthenyl-1,2,3-thiadiazol-3-in-5-yliden)-1-phenylharnstoff | 500 | 96 |
| 3-(2-Methoxymethyl-1,2,3-thiadiazol-3-in-5-yliden)-1-phenylharnstoff | 500 | 100 |
| 3-(2-Äthyl-1,2,3-thiadiazol-3-in-5-yliden)-1-methyl-1-phenylharnstoff | 500 | 100 |

-22-

BEISPIEL 22

Junge Baumwollpflanzen im Stadium von 5 bis 6 entwickelten Laubblättern wurden wie im Beispiel 21 behandelt und nach 7 Tagen ausgewertet, das heißt der Prozentsatz abgefallener Blätter festgestellt:

| Erfindungsgemäße Verbindungen | Dosis in g Wirkstoff/ha | Entblätterung in % |
|---|---|---|
| 3-(2-Methyl-1,2,3-thiadiazol-3-in-5-yliden)-1-phenylharnstoff | 40 | 90 |
| 3-(2-Äthyl-1,2,3-thiadiazol-3-in-5-yliden)-1-phenylharnstoff Hydrochlorid | 40 | 90 |
| 3-(2-Äthyl-1,2,3-thiadiazol-3-in-5-yliden)-1-phenylharnstoff | 40 | 95 |
| 3-(2-Propyl-1,2,3-thiadiazol-3-in-5-yliden)-1-phenylharnstoff | 40 | 85 |
| 3-(2-Butyl-1,2,3-thiadiazol-3-in-5-yliden)-1-phenylharnstoff | 40 | 85 |
| 3-(2-Methyl-1,2,3-thiadiazol-3-in-5-yliden)-1-phenylharnstoff Hydrochlorid | 40 | 80 |
| 3-(2-Propyl-1,2,3-thiadiazol-3-in-5-yliden)-1-phenylharnstoff Hydrochlorid | 40 | 85 |
| 3-(2-Butyl-1,2,3-thiadiazol-3-in-5-yliden)-1-phenylharnstoff Hydrochlorid | 40 | 84 |
| 3-(2-Isopropyl-1,2,3-thiadiazol-3-in-5-yliden)-1-phenylharnstoff | 40 | 62 |
| 3-(2-(2-Propenyl)-1,2,3-thiadiazol-3-in-5-yliden)-1-phenylharnstoff | 40 | 52 |
| 3-(2-Äthenyl-1,2,3-thiadiazol-3-in-5-yliden)-1-phenylharnstoff | 40 | 62 |
| **Vergleichsmittel gemäß DE-OS 2214632 und 2506690** | | |
| 1-Phenyl-3-(1,2,3-thiadiazol-5-yl)-harnstoff | 40 | 38 |



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 23

Junge Baumwollpflanzen im Stadium von 5 bis 6 entwickelten Blättern wurden wie im Beispiel 21 behandelt und im Gewächshaus bei 20 bis 25°C gehalten. Fünf Tage und drei Wochen nach der Applikation wurde der Prozentsatz abgefallener Blätter festgestellt. Die ermittelten Werte lassen die rasche Entblätterung der Pflanzen durch die erfindungsgemäßen Verbindungen erkennen.

| Erfindungsgemäße Verbindungen | Dosis in g Wirkstoff/ha | Entblätterung in % nach 5 Tagen | 22 Tagen |
|---|---|---|---|
| 3-(2-Äthyl-1,2,3-thiadiazol-3-in-5-yliden)-1-phenylharnstoff Hydrochlorid | 80 | 57 | 100 |
| 3-(2-Äthyl-1,2,3-thiadiazol-3-in-5-yliden)-1-phenylharnstoff | 80 | 38 | 100 |
| 3-(2-Propyl-1,2,3-thiadiazol-3-in-5-yliden)-1-phenylharnstoff | 80 | 43 | 100 |
| 3-(2-Propyl-1,2,3-thiadiazol-3-in-5-yliden)-1-phenylharnstoff Hydrochlorid | 80 | 33 | 100 |
| **Vergleichsmittel gemäß DE-OS 2214632 und 2506690** | | | |
| 1-Phenyl-3-(1,2,3-thiadiazol-5-yl)-harnstoff | 80 | 10 | 90 |

-24-

BEISPIEL 24

Samen von Amaranthus caudatus wurden in einer Petrischale mit einer Scheibe Filterpapier und 5 ml Phosphatpuffer (1 mM, pH 6,8) zum Keimen gebracht. Hierzu wurden 30 Samen gleichmäßig auf dem getränkten Filterpapier verteilt. Die Keimlösung enthielt den formulierten Wirkstoff und 1 g Tyrosin/l. Nach dem Auftragen der Samen wurde die Petrischale ins Dunkle gestellt (25°C, 70 % relative Luftfeuchtigkeit).

Nach vier Tagen wurde die Rotfärbung der Keimlinge als Anzeichen für einen cytokininartigen Effekt bonitiert:

0 = wie Kontrolle
1 = schwache Rotfärbung
2 = mittlere Rotfärbung
3 = starke Rotfärbung
4 = sehr starke Rotfärbung, Wurzeln verkürzt, höchste Cytokininaktivität

| Erfindungsgemäße Verbindungen | Molarität der Prüflösung (M) | Boniturnote |
|---|---|---|
| 3-(2-Äthyl-1,2,3-thiadiazol-3-in-5-yliden)-1-phenylharnstoff Hydrochlorid | $10^{-7}$ | 3 |
| | $10^{-8}$ | 4 |
| 3-(2- Äthyl-1,2,3-thiadiazol-3-in-5-yliden)-1-phenylharnstoff | $10^{-7}$ | 4 |
| | $10^{-8}$ | 3 |
| 3-(2-Propyl-1,2,3-thiadiazol-3-in-5-yliden)-1-phenylharnstoff | $10^{-7}$ | 3 |
| | $10^{-8}$ | 0 |
| 3-(2-Isopropyl-1,2,3-thiadiazol-3-in-5-yliden)-1-phenylharnstoff | $10^{-7}$ | 4 |
| | $10^{-8}$ | 4 |

Es zeigte sich, daß die erfindungsgemäßen Verbindungen noch in sehr geringer Konzentration deutliche Cytokinineffekte hervorriefen.

-25-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 25

Sojapflanzen wurden im Nachauflaufverfahren mit den erfindungsgemäßen Substanzen behandelt und im Gewächshaus gezogen. Die Konzentration des Wirkstoffes betrug umgerechnet 10 g/ha. Nach 6 Tagen wurden aus den Primärblättern Scheiben ausgestanzt, das darin enthaltene Chlorophyll wurde extrahiert und photometrisch bestimmt. Der so erhaltene Chlorophyllgehalt wurde in Relation zur Kontrolle gesetzt.

| Erfindungsgemäße Verbindungen | Chlorophyllanreicherung im Vergleich zur Kontrolle(./.) |
|---|---|
| 3-(2-Decyl-1,2,3-thiadiazol-3-in-5-yliden)-1-phenylharnstoff | 112,6 |
| 3-(2-(2-Acetoxyäthyl)-1,2,3-thiadiazol-3-in-5-yliden)-1-phenylharnstoff | 124,9 |
| 3-(2-(2-Chloräthyl)-1,2,3-thiadiazol-3-in-5-yliden)-1-phenylharnstoff | 109,9 |
| 3-(2-Benzyl-1,2,3-thiadiazol-3-in-5-yliden)-1-phenylharnstoff | 126,8 |
| 1-Phenyl-3-(2-(2-propinyl)-1,2,3-thiadiazol-3-in-5-yliden)-harnstoff | 129,4 |
| 3-(2-Propyl-1,2,3-thiadiazol-3-in-5-yliden)-1-phenylharnstoff | 117,2 |
| Kontrolle | 100.0 |

Die erfindungsgemäßen Substanzen verursachten also schon kurze Zeit nach der Applikation eine deutliche Chlorophyllanreicherung in den Blättern.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 26

Weizensamen wurden in Glasstutzen 4 Stunden lang in einer wäßrigen Prüflösung gequollen. Danach wurde in die Stutzen eine wäßrige Lösung von Polyäthylenglykol 6000 gegeben, so daß die Lösung in dem Stutzen 22,5 % Polyäthylenglykol enthielt. Nun wurden die Stutzen mit dem Deckel einer Petrischale abgedeckt, in eine Klimakammer mit 25°C und 70% relativer Luftfeuchte gestellt und 12 Stunden pro Tag beleuchtet. Nach einer Woche wurde der Keimungsverlauf registriert. Hierzu wurde die Länge der Triebe gemessen (beziehungsweise der Koleoptile oder Koleoptile und 1 Laubblatt). Die nachfolgende Tabelle enthält die Ergebnisse eines Versuchs, in dem nach dem Zugeben des Polyäthylenglykols die Prüfsubstanzen 0,01, 0,001 und 0,0001 %ig enthalten waren.

| Erfindungsgemäße Verbindungen | Konzentration der Verbindungen in % | Sproßlänge in cm |
|---|---|---|
| 1-Phenyl-3-(2-(2-propinyl)-1,2,3-thiadiazol-3-in-5-yliden)-harnstoff | 0.01 | 3,8 |
| | 0.001 | 1,9 |
| | 0,0001 | 2.0 |
| 3-(2-Benzyl-1,2,3-thiadiazol-3-in-5-yliden)-1-phenylharnstoff | 0.01 | 5.1 |
| | 0.001 | 5.2 |
| | 0.0001 | 1.1 |
| 3-(2-(2-Chloräthyl)-1,2,3-thiadiazol-3-in-5-yliden)-1-phenylharnstoff | 0.01 | 4.0 |
| | 0.001 | 3.5 |
| | 0.0001 | 2.0 |
| Kontrolle | - | 0.2 |

Es zeigte sich, daß die Kontrolle - die nur in Leitungswasser vorgequollen war - durch das Osmoticum stark im Wachstum gehemmt war.

Die Keimlinge, die mit den erfindungsgemäßen Substanzen vorbehandelt waren, wuchsen trotz des osmotischen Stresses sehr viel rascher als die unbehandelte Kontrolle.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

## BEISPIEL 27

Sojapflanzen wurden im Primäblatt-Stadium mit den erfindungsgemäßen Substanzen behandelt. Dabei wurden umgerechnet 10 g und 50 g Aktivsubstanz je ha appliziert.

Die Pflanzen wurden bei 25°C und 70 % relativer Luftfeuchte in der Klimakammer kultiviert.
Nach 4 Wochen wurde die Anzahl gebildeter Blüten und Hülsenansätze erfaßt. Diese Zahl wurde in Relation zur Kontrolle gesetzt. Außerdem wurde der Verzweigungsgrad der Pflanzen in einer Boniturskala von 0 - 4 festgehalten, dabei bedeutet
0 = Verzweigung wie bei der Kontrolle
1 = schwache Förderung der Verzweigung
2 = mittlere Förderung der Verzweigung
3 = starke Förderung der Verzweigung
4 = Verzweigung im Vergleich zur Kontrolle sehr stark gefördert

Wuchsreduktionen wurden ebenfalls im Verhältnis zur Kontrolle ermittelt.

| Erfindungsgemäße Verbindungen | Konzentration Wirkstoff/ha | Wuchsreduktion in % | Blüten und Hülsen in % | Verzweigung |
|---|---|---|---|---|
| 3-(2-(2-Acetoxyäthyl)-1,2,3-thiadiazol-3-in-5-yliden)-1-phenylharnstoff | 10 | 25 | 119 | 4 |
| | 50 | 50 | 108 | 4 |
| Kontrolle | - | 0 | 100 | 0 |

Die erfindungsgemäßen Substanzen griffen stark in die Stoffwechselphysiologie von Sojapflanzen ein, dies bewirkte eine Steigerung der gebildeten Blütenzahl, eine intensive Verzweigung und eine Reduktion des Wuchses.

-28-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

## PATENTANSPRÜCHE

1. 1,2,3-Thiadiazol-3-in-5-yliden-harnstoffderivate der allgemeinen Formel

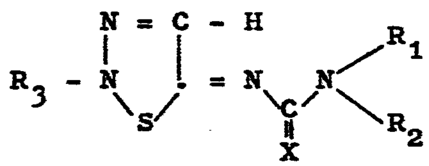

I

in der

$R_1$ Wasserstoff oder einen gegebenenfalls ein- oder mehrfach durch Sauerstoff- oder Schwefelatome unterbrochenen $C_1$-$C_4$-Alkylrest,

$R_2$ einen gegebenenfalls ein- oder mehrfach durch Sauerstoff- oder Schwefelatome unterbrochenen $C_1$-$C_4$-Alkylrest, einen gegebenenfalls ein- oder mehrfach durch Alkyl substituierten $C_3$-$C_8$-Cycloalkylrest, einen gegebenenfalls ein- oder mehrfach durch Alkyl und/oder Halogen und/oder Alkylthio und/oder Alkoxy und/oder Trifluormethyl und/oder die Nitrogruppe substituierten aromatischen Kohlenwasserstoffrest oder einen gegebenenfalls substituierten heterocyclischen, mindestens ein N-Atom enthaltenen, Kohlenwasserstoffrest oder $R_1$ und $R_2$ gemeinsam mit dem N-Atom die Morpholino-, Piperidino- oder Pyrrolidinogruppe,

$R_3$ einen gegebenenfalls substituierten $C_1$-$C_{10}$-Alkylrest, einen $C_2$-$C_6$-Alkenyl- oder $C_3$-$C_6$-Alkinylrest oder einen gegebenenfalls substituierten Aryl-$C_1$-$C_2$-alkylrest und

X ein Sauerstoff- oder ein Schwefelatom

bedeuten sowie deren Säureadditionssalze mit anorganischen und organischen Säuren.

2. 1,2,3-Thiadiazol-3-in-5-yliden-harnstoffderivate gemäß Anspruch 1, worin

$R_1$ Wasserstoff, Methyl, Äthyl, Propyl, Isopropyl oder Butyl,

$R_2$ Methyl, Äthyl, Propyl, Cyclopentyl, Cyclohexyl, Methylcyclohexyl, Phenyl, Halophenyl, Nitrophenyl, Trifluormethylphenyl, Methoxyphenyl, oder Pyridyl,

-29-

$R_3$ Methyl, Äthyl, Propyl, Isopropyl, n-Butyl, Isobutyl, sec-Butyl, tert-Butyl, Pentyl, Hexyl, Heptyl, Octyl, Nonyl, Decyl, 3,3-Dimethylpropyl, Chlormethyl, Fluormethyl, 2-Chloräthyl, 2-Hydroxyäthyl, 2-Methylsulfonyloxyäthyl, 2-Acetoxyäthyl, Methoxymethyl, 2-Methoxyäthyl, 2-Äthoxyäthyl, 2-Phenoxyäthyl, 2-(2,4-Dichlorphenoxy)-äthyl, 2-(4-Chlorphenoxy)-äthyl, 2-Dimethylamino-äthyl, 3-Chlorpropyl, 3-Methoxypropyl, (2-Methyl-1,3-dioxolan-2-yl)-methyl, 3-Dimethylaminopropyl, 3-Phenoxypropyl, 2,2-Dichlorcyclopropylmethyl, Äthenyl, 2-Propenyl, 3-Methyl-2-buten-1-yl, 2-Methyl-1-propen-3-yl, Hexenyl, Heptenyl, Octenyl, 2-Propinyl, Butinyl, Pentinyl, Hexinyl, Benzyl, 2-Fluorbenzyl, 4-Fluorbenzyl, 2-Chlorbenzyl, 2-Brombenzyl, 3-Brombenzyl, 4-Brombenzyl, 2,4-Dichlorbenzyl, 2,6-Dichlorbenzyl, 3,4-Dichlorbenzyl, 2-Methylbenzyl, 3-Methylbenzyl, 4-Methylbenzyl, 2-Nitrobenzyl, 3-Nitrobenzyl, 4-Nitrobenzyl, 2-Trifluormethylbenzyl, 3-Trifluormethylbenzyl, 4-Trifluormethylbenzyl, 2-Methoxybenzyl, 3-Methoxybenzyl, 4-Methoxybenzyl, 2-Äthoxybenzyl, 3-Äthoxybenzyl, 4-Äthoxybenzyl, 2-Propoxybenzyl, 3-Propoxybenzyl, 4-Propoxybenzyl, 2-Butoxybenzyl, 3-Butoxybenzyl, 4-Butoxybenzyl, 2-Methylthiobenzyl, 3-Methylthiobenzyl, 4-Methylthiobenzyl, 2-Äthylthiobenzyl, 3-Äthylthiobenzyl, 4-Äthylthiobenzyl, 2-Butylthiobenzyl, 3-Butylthiobenzyl oder 4-Butylthiobenzyl und

X ein Sauerstoff- oder ein Schwefelatom bedeuten sowie deren Säureadditionssalze mit anorganischen und organischen Säuren.

3. 3-(2-Propyl-1,2,3-thiadiazol-3-in-5-yliden)-1-phenylharnstoff

4. 3-(2-Propyl-1,2,3-thiadiazol-3-in-5-yliden)-1-phenylharnstoff, Hydrochlorid

-30-

5. 3-(2-Äthyl-1,2,3-thiadiazol-3-in-5-yliden)-1-phenylharnstoff, Hydrochlorid
6. 3-(2-Isopropyl-1,2,3-thiadiazol-3-in-5-yliden)-1-phenylharnstoff
7. 1-Phenyl-3-(2-(2-propinyl)-1,2,3-thiadiazol-3-in-5-yliden)-harnstoff
8. 3-(2-(2-Acetoxyäthyl)-1,2,3-thiadiazol-3-in-5-yliden)-1-phenylharnstoff
9. 3-(2-Äthyl-1,2,3-thiadiazol-3-in-5-yliden)-1-phenyl-harnstoff

-31-

10. Verfahren zur Herstellung von 1,2,3-Thiadiazol-in-5-yliden-harnstoffderivaten gemäß Ansprüchen 1 bis 9, dadurch gekennzeichnet, daß man

A) Metallverbindungen der allgemeinen Formel

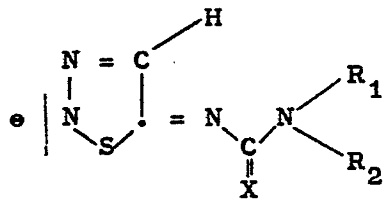 $B^{\oplus}$ II

mit Verbindungen der allgemeinen Formel

$R_3 - Z$ III,

gegebenenfalls in Gegenwart eines Lösungsmittels und gegebenenfalls in Gegenwart eines Katalysators, reagieren läßt oder

B) (1,2,3-Thiadiazol-5-yl)-harnstoffe der allgemeinen Formel

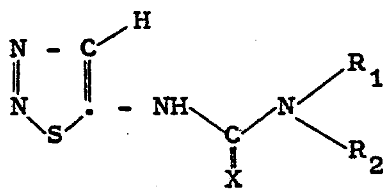 IV

mit Verbindungen der allgemeinen Formel

$R_3 - Z$ III

gegebenenfalls in Gegenwart von säurebindenden Mitteln, sowie gegebenenfalls in Gegenwart eines geeigneten Katalysators umsetzt, worin $R_1$, $R_2$, $R_3$ und X die oben genannte Bedeutung haben, Z ein Halogenatom oder den Rest $R_3OSO_2-O$ und B ein einwertiges Metalläquivalent bedeuten.

11. Wuchsregulatorische und entblätternde Mittel, gekennzeichnet durch einen Gehalt an mindestens einer Verbindung gemäß den Ansprüchen 1 bis 9.

12. Mittel gemäß Anspruch 11 in Mischung mit Träger und/oder Hilfsstoffen.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101, Bankleitzahl 100 100 10
Formular-Nr. 1439 4